# EUROPÄISCHE PATENTANMELDUNG

(11) **EP 0 711 739 A1**
(43) Veröffentlichungstag der Anmeldung: **15.05.1996**
(21) Anmeldenummer: 95117128.9
(22) Anmeldetag: 31.10.1995
(51) Int. Cl.: C05G 3/00, B01J 2/30

(54) **Mittel zur Herstellung von nichtstaubenden, nicht zusammenbackenden Düngemitteln**

(30) Priorität: 08.11.1994 DE 4439837; 14.07.1995 DE 19525730
(71) Anmelder: HOECHST AKTIENGESELLSCHAFT, D-65929 Frankfurt am Main (DE)
(72) Erfinder: Gatter, Erich, Dr., D-84556 Kastl (DE); Vybiral, Reinhard, Dr., D-84504 Burgkirchen (DE); Pedain, Klaus-Ulrich, D-63128 Steinberg (DE); Witte, Christian, D-60345 Frankfurt (DE); Wehle, Detlef, Dr., D-65611 Brechen (DE)

(57) **Zusammenfassung**

Gegenstand der Erfindung sind zur Herstellung von nichtstaubenden und nichtzusammenbackenden Düngemitteln geeignete Mischungen, enthaltend a) synthetische und/oder natürliche Öle und b) N-Alkyl-N-Alkanolamin-Fettsäureester der allgemeinen Formel I, 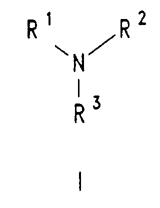
worin R¹ für einen aliphatischen oder Hydroxyalkylrest mit 1 bis 6 Kohlenstoffatomen oder für R³,
R² für R³,
R³ für 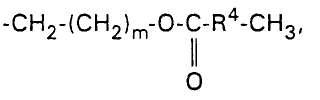
m für eine ganze Zahl von 1 bis 5,
R⁴ für einen geradkettigen oder verzweigten Alkyl- oder Alkenylrest von 8 bis 24 C-Atomen steht,
und gegebenenfalls zusätzlich oder gegebenenfalls anstelle von a) Fettsäureverbindungen der allgemeinen Formel II 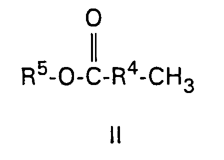
worin R⁵ für einen aliphatischen oder Hydroxyalkylrest mit 1 bis 6 Kohlenstoffatomen oder für Wasserstoff steht und R⁴ die vorstehend beschriebene Bedeutung hat, und gegebenenfalls weitere Hilfsmittel. Gegenstand der Erfindung ist ferner die Verwendung dieser Mischung zur Herstellung nichtstaubender und nichtzusammenbackender Düngemittel sowie Düngemittel, die mit dieser Mischung behandelt worden sind.

## Beschreibung

Gegenstand der Erfindung sind zur Herstellung von nichtstaubenden und nichtzusammenbackenden Düngemitteln geeignete Mischungen, sowie deren Verwendung zur Herstellung derselben.

Feste mineralische Düngemittel, die wasserlösliche Substanzen enthalten, neigen bei der Lagerung zum Zusammenbacken, unabhängig davon, ob sie als Granulate, Prills, Kristallisate oder Pulver vorliegen. Hierdurch wird die Handhabung erheblich erschwert. Es ist bekannt, Düngemittel gegen das Zusammenbacken dadurch zu schützen, daß sie mit Puderstoffen umhüllt werden. Die Haftfestigkeit der Puderstoffe ist jedoch begrenzt und es kommt beim Ein- und Ausspeichern der Düngemittel zu einer Staubbelästigung.

DE-A 1592 572 beschreibt ein Verfahren zur Vermeidung des Zusammenbackens und des Staubens, wobei Salze und Düngemittel mit Polyethylenwachs umhüllt werden. DE-A 1905 834 offenbart ein ähnliches Verfahren, wobei auch Polypropylenwachs mit einem mittleren Molekulargewicht von 500 bis 10000 und eine oberflächenaktive Substanz zur Umhüllung verwendet werden.

Diese Verfahren sind jedoch nicht allgemein anwendbar. Einerseits muß wegen des relativ hohen Schmelzpunktes der vorgeschlagenen Wachse (≧ 90°C) die Temperatur des Düngemittels während der Umhüllung mit dem Wachs mindestens 80°C, vorzugsweise jedoch bis 30°C oberhalb des Schmelzpunktes liegen. Das Verfahren ist also für Düngemittel, bei deren Herstellung, nach dem Absieben von Unterkorn und Überkorn, das Fertigkorn mit einer Temperatur unterhalb von 80°C anfällt, nur dann anwendbar, wenn das zu umhüllende

Düngemittel auf die erforderliche Temperatur aufgeheizt wird. Hierdurch wird das Verfahren unwirtschaftlich. Andererseits können die Düngemittel in unterschiedlicher Qualität anfallen, so daß es in einigen Fällen, in denen die Düngemittel in besonders starkem Maße zum Zusammenbacken neigen, nicht möglich ist, eine hinreichend wirksame verbackungshemmende Umhüllung mit den vorbekannten Polyalkylenwachsen und oberflächenaktiven Substanzen zu erhalten.

Es ist weiterhin bekannt, Düngemittel mit Oxalkylaten als oberflächenaktive Stoffe zu umhüllen (FR-A 1 100 686, BE-A 606734, GB-A 743 600 und 800 794). Die wasserabweisende Wirkung dieser Zusätze muß durch zusätzliche Puderstoffe erhöht werden, die jedoch die schon erwähnte Staubbelästigung verursachen.

Ferner wurde die Umhüllung von Düngemitteln mit Oxalkylaten und Tensiden beschrieben (DE-A 2109199). Die dabei verwendeten Fettamine sind jedoch aus ökologischen Gesichtspunkten nicht unbedenklich.

Aufgabe der Erfindung ist es, Mittel zur Herstellung von nichtstaubenden und nichtzusammenbackenden Düngemitteln bereitzustellen, die die vorstehend beschriebenen Nachteile nicht aufweisen.

Diese Aufgabe wird gelöst durch Mischungen, enthaltend a) synthetische und/oder natürliche Öle und b) N-Alkyl-N-Alkanolamin-Fettsäureester der allgemeinen Formel I, 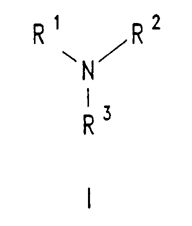
worin R¹ für einen aliphatischen oder Hydroxyalkylrest mit 1 bis 6 Kohlenstoffatomen oder für R³,
R² für R³,
R³ für 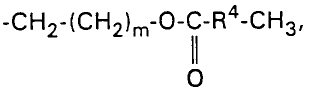
m für eine ganze Zahl von 1 bis 5,
R⁴ für einen geradkettigen oder verzweigten Alkyl- oder Alkenylrest von 8 bis 24 C-Atomen steht,
und gegebenenfalls zusätzlich oder gegebenenfalls anstelle von a) Fettsäureverbindungen der allgemeinen Formel II 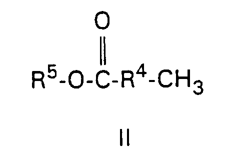
worin R⁵ für einen aliphatischen oder Hydroxyalkylrest mit 1 bis 6 Kohlenstoffatomen oder für Wasserstoff steht und R⁴ die vorstehend beschriebene Bedeutung hat, und gegebenenfalls weitere Hilfsmittel.

Bei dem aliphatischen Rest R¹ und R⁵ kann es sich um einen geradkettigen oder verzweigten, gegebenenfalls auch cycloaliphatischen Rest handeln. Beispiele hierfür sind Methyl, Ethyl, Propyl, Butyl, tert.-Butyl, Cyclobutyl, Cyclopentyl, Cyclohexyl. Für den Rest R⁵ wird bevorzugt Wasserstoff eingesetzt.

Von den Verbindungen der Formel II sind Stearinsäure, Ölsäure und teilhydrierte Talgfettsäure von besonderem Interesse.

Weitere Hilfsmittel können beispielsweise oxalkylierte Fettalkohole mit 12 bis 22, bevorzugt 16 bis 18 Kohlenstoffatomen sein, die 8 bis 60, bevorzugt 20 bis 50 Alkylenoxidgruppen enthalten.

Bei dem Hydroxyalkylrest handelt es sich beispielsweise um Methanol, Ethanol, Propanol, Isopropanol, Butanol oder tert.-Butanol.

Vorzugsweise werden in den erfindungsgemäßen Mischungen Verbindungen der allgemeinen Formel I eingesetzt, worin
R¹ für einen Alkyl- oder Hydroxyalkylrest mit 1 bis 4 Kohlenstoffatomen,
R² für R³,
R³ für 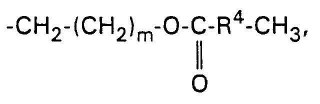
m für eine ganze Zahl von 1 bis 4, insbesondere für 1,
R⁴ für einen Alkyl- oder Alkenylrest mit 14 bis 20, insbesondere 16 C-Atomen steht.

Insbesondere bevorzugt werden in den erfindungsgemäßen Mischungen Verbindungen der Formel I eingesetzt, worin
R¹ für Methyl,
R² für R³,
R³ für 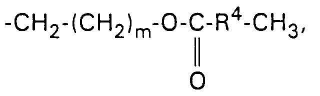
m für die Zahl 1,
R⁴ für einen Alkyl- oder Alkenylrest mit 16 C-Atomen steht.

Die vorstehend beschriebenen Vorteile erreicht man auch mit Mischungen, enthaltend Verbindungen der allgemeinen Formel I, worin
R¹ für -CH₂-CH₂-OH,
R² für R³,
R³ für 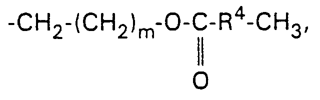
m für die Zahl 1,
R⁴ für einen Alkyl- oder Alkenylrest mit 16 C-Atomen steht.

Bei den natürlich vorkommenden Ölen handelt es sich beispielsweise um Soja-, Sonnenblumen-, Oliven-, Lein-, Rüb-, Sesam-, Erdnuß-, Palm-, Reis-, Cocos-, Tall-, Traubenkern- oder Weizenkeimöl und Mischungen derselben. Die Verwendung dieser nachwachsenden Rohstoffe stellt einen weiteren Vorteil der Erfindung dar.
Daneben können auch Gemische von natürlich vorkommenden oder synthetisch hergestellten freien Fettsäuren, sowie Gemische von Alkylestern, bevorzugt C₁-C₆-Alkylestern, insbesondere von Methylestern, dieser Fettsäuren verwendet werden. Beispiele für mit gutem Erfolg einsetzbare Fettsäuren sind Capryl-, Caprin-, Laurin-, Myristin-, Palmitin-, Stearin-, Arachin-, Öl- oder Erucasäure.

Die erfindungsgemäßen Mittel enthalten im allgemeinen bis zu 50 Gew.-%, vorzugsweise zwischen 10 und 40 Gew.-% von Verbindungen der allgemeinen Formel I und bis zu 90 Gew.-%, vorzugsweise zwischen 55 und 85 Gew.-% Lösungsmittel auf Basis natürlicher oder synthetischer Öle und gegebenenfalls zusätzlich oder anstelle der Lösungsmittel langkettige Fettsäuren der Formel II oder deren Alkylester, sowie gegebenenfalls weitere Hilfsmittel bis zu 30 Gew.-%.

Die vorstehend beschriebenen Mischungen sind zur Herstellung von nichtstaubenden und nichtzusammenbackenden Düngemitteln geeignet. Ihr Vorteil liegt in der besseren biologischen Abbaubarkeit, so daß eine Anreicherung in den Ackerböden vermieden werden kann. Im OECD-Test nach Europa-Norm (EEC 84/449) erreichen die erfindungsgemäßen Mischungen Abbauraten von über 80 %, während die bisher bekannten Mittel nur Werte von etwa 30 % erreichen.

Weiterer Gegenstand der vorliegenden Erfindung ist die Verwendung der erfindungsgemäßen Mischungen zur Herstellung von nichtstaubenden und nichtzusammenbackenden Düngemitteln.

Die Behandlung der Düngemittel mit den erfindungsgemäßen Mischungen erfolgt in bekannter Weise, wie in DE-A 2109 199, DE-A 1592 796, oder an anderer Stelle beschrieben.

Ein weiterer Gegenstand der vorliegenden Erfindung sind Düngemittel, die mit den erfindungsgemäßen Mischungen behandelt worden sind. Als Düngemittel seien beispielsweise genannt Ammoniumnitrat, Kalksalpeter, Harnstoff, Kaliumchlorid, oder Mehrnährstoffdünger wie z.B. NPK-Dünger. Weitere geeignete Düngemittel finden sich bei Ullmann, Enzyklopädie der Technischen Chemie, Bd. 10, S. 220.

Die erfindungsgemäßen Mischungen werden üblicherweise in einer Menge von 0,1 bis 3 kg/t, vorzugsweise von 0,5 bis 1,8 kg/t verwendet, bezogen auf den eingesetzten Dünger.

Die nachfolgenden Beispiele belegen die Erfindung, ohne sie zu beschränken.

### Beispiele

Die Ergebnisse der Wirksamkeit der erfindungsgemäßen Mittel zur Verminderung der Verbackungsneigung und des Staubanfalls an verschiedenen Düngemitteln sind in der Tabelle zusammengefaßt.

Die Behandlung der Düngemittel mit den erfindungsgemäßen Mitteln wurde in einer als Trommel dienenden 5-l-Aluminiumkanne durchgeführt. Sie konnte von außen zum Aufheizen oder zum Abkühlen mit Heiß- oder Kaltluft umspült werden. In die Aluminiumkanne waren von außen vier etwa 3 mm tiefe Rillen hineingedrückt, die im Innenraum die Wirkung von Mitnehmerleisten (Hubleisten) hervorriefen. Die Aluminiumkanne rotierte bei einem Winkel von etwa 5° zwischen Längsachse und Horizontalen mit einer Geschwindigkeit von bis zu 50 Umdrehungen pro Minute. Die Antibackmittel wurden in flüssiger Form auf die Düngemittelprobe aufgetragen und bei einer Rolldauer von 10 Minuten gleichmäßig verteilt. Nach Beendigung der Behandlung wurde die Probe in luftdicht verschlossenen Flaschen zwischengelagert. Die Aluminiumkanne wurde nach jedem Versuch von noch eventuell anhaftenden Behandlungsstoffen gereinigt.

Der Verbackungstest wurde nach der in der Literatur beschriebenen Vorschrift durchgeführt (Chemie-Ingenieur-Technik, 40. Jahrgang, 1968, Heft 4, S. 191). 300 g des behandelten Düngemittels wurden in die fünf Ringpaare eines Verbackungstestrohres mit Zwischenstempel gefüllt und in einem Luftthermostaten 6 Tage lang bei einer Temperatur von 45°C oder bei 35°C-Raumtemperatur bzw. 32°C-Raumtemperatur (Temperaturwechsel in 4 Stunden-Intervallen) mit 20 kg belastet. Der spezifische Druck beträgt bei Verwendung von Ringen mit einem inneren Durchmesser von 39 mm 1,6 kg/cm² und entspricht etwa dem Druck, dem ein Düngemittel in einer Lagerhalle mit einer Schütthöhe von 15 m ausgesetzt ist. Nach der Aufhebung der Druckbelastung wird das Verbackungstestrohr aus dem Lufthermostat genommen und 20 Stunden bei Raumtemperatur gelagert, um eine hinreichende und gleichmäßige Abkühlung sicherzustellen. Aus dem luftdicht verschlossenen Verbackungstestrohr werden sodann die in Ringpaaren gefaßten und zusammengebackenen Proben auf den Grad des Zusammenbackens getestet. Hierzu wird der eine Ring in eine Halterung geschoben und der andere Ring unmittelbar neben der Berührungszone beider Ringe zunehmend belastet. Die Belastungszunahme erfolgt von 0 auf 150 N (Newton), von 0 auf 300 N und von 0 auf 450 N in jeweils 37 Sekunden. Bestimmt wird die Last, die zum Abscheren des zweiten Ringes erforderlich ist. Sie wird als Scherlast in N angegeben. Die Menge an Staub wurde mit einer Filter-Fritte (Typ G3, Porengröße 16-40 µ) bestimmt, durch die die staubhaltige Luft gesaugt wurde.

Die Ergebnisse der Versuchsreihen sind in der Tabelle wie folgt dargestellt. Die beiden ersten Versuche jeder Reihe stellen Vergleichsproben dar, die entweder unbehandelt (Blindwert) oder mit einem Referenzmittel hergestellt wurden. Als Maß für die Neigung des Zusammenbackens ist die Scherlast angegeben. Die Verbackungsneigung wird in Prozent, bezogen auf ein unbehandeltes Düngemittel (= 100 % Verbackungsneigung), wiedergegeben.

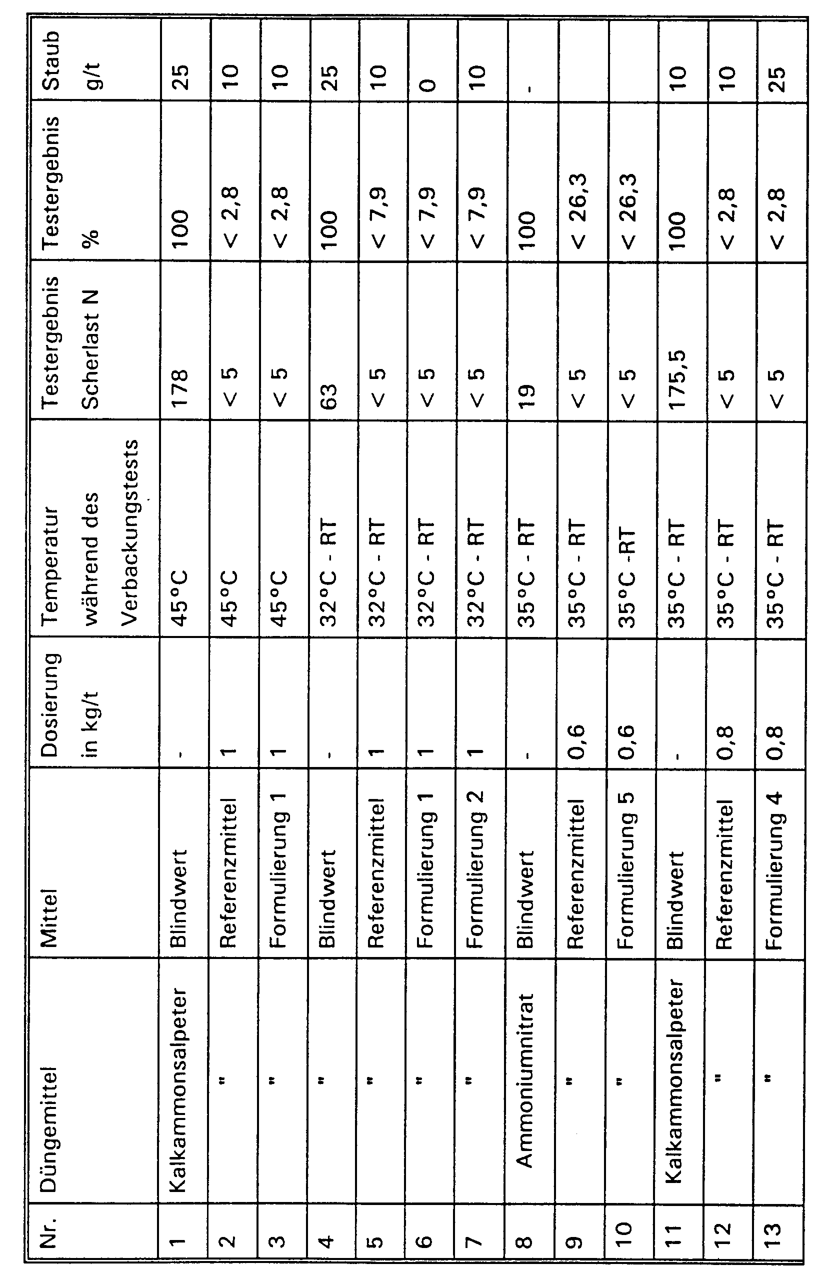

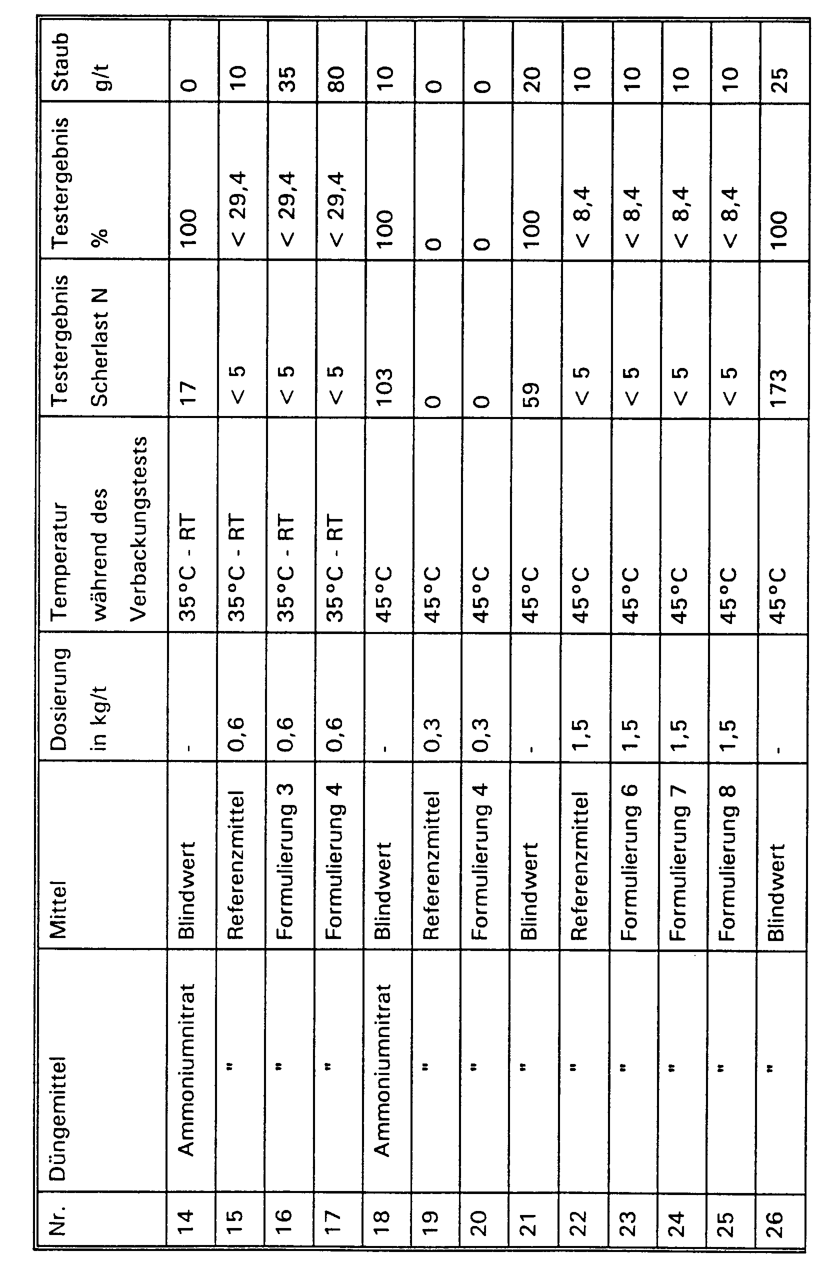

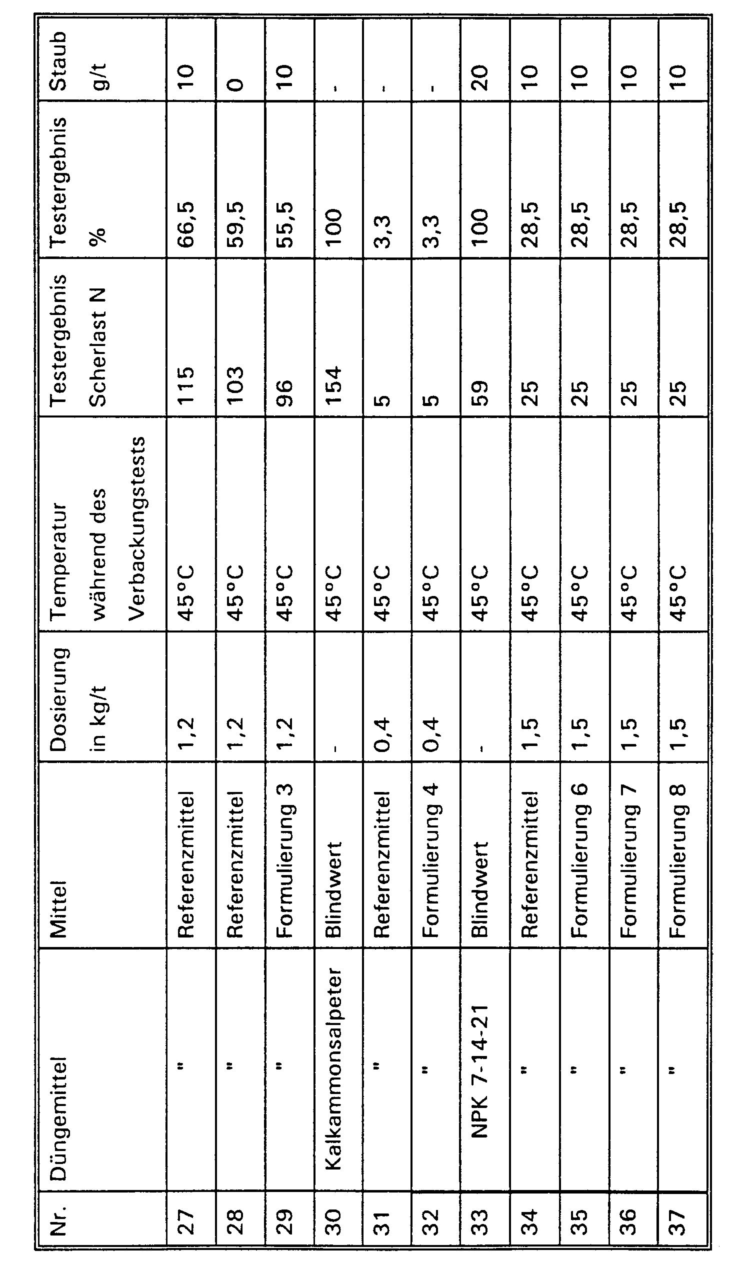

Eingesetzte erfindungsgemäße Mittel:
- Formulierung 1.): 30 % N-Methyl-di-N-ethanolamin-stearinsäureester (A) + 70 % Sojaölfettsäure
- Formulierung 2.): 15 % (A) + 85 % Resines 27 (Polymere des Methylricinoleats und von Methylester C 18 und C 20)
- Formulierung 3.): 15 % (A) + 75 % Sojaöl + 10 % Stearinsäure
- Formulierung 4.): 15 % (A) + 75 % Rüböl + 10 % Stearinsäure
- Formulierung 5.): 15 % (A) + 75 % Resines 27 + 10 % Stearinsäure
- Formulierung 6.): 15 % (A) + 56 % Sojaölfettsäure + 29 % ®Genapol T-400 (Talgfettalkohol mit 40 EO)
- Formulierung 7.): 15 % (A) + 56 % Edenor NRA (ungehärtete Rübölfettsäure) + 29 % ®Genapol T-400
- Formulierung 8.): 15 % (A) + 56 % Resines 27 + 29 % ®Genapol T-400
Die verwendeten Referenzmittel basieren auf Fettaminen.

## Patentansprüche

1. Zur Herstellung von nichtstaubenden und nichtzusammenbackenden Düngemitteln geeignete Mischung, enthaltend
a) synthetische und/oder natürliche Öle und
b) N-Alkyl-N-Alkanolamin-Fettsäureester der allgemeinen Formel I, 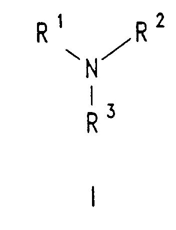 worin
R¹ für einen aliphatischen oder Hydroxyalkylrest mit 1 bis 6 Kohlenstoffatomen oder für R³,
R² für R³,
R³ für 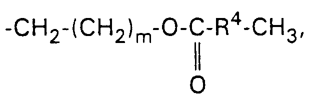 m für eine ganze Zahl von 1 bis 5,
R⁴ für einen geradkettigen oder verzweigten Alkyl- oder Alkenylrest von 8 bis 24 C-Atomen steht,
und gegebenenfalls zusätzlich oder gegebenenfalls anstelle von a) Fettsäureverbindungen der allgemeinen Formel II 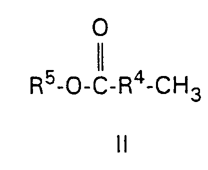 worin R⁵ für einen aliphatischen oder Hydroxyalkylrest mit 1 bis 6 Kohlenstoffatomen oder für Wasserstoff steht und R⁴ die vorstehend beschriebene Bedeutung hat,
und gegebenenfalls weitere Hilfsmittel.

2. Mischung nach Anspruch 1, enthaltend Verbindungen der allgemeinen Formel I, worin
R¹ für einen Alkylrest mit 1 bis 4 Kohlenstoffatomen,
R² für R³,
R³ für 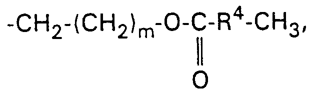 m für eine ganze Zahl von 1 bis 4, insbesondere für 1,
R⁴ für einen Alkyl- oder Alkenylrest mit 8 bis 24 C-Atomen steht.

3. Mischung nach den Ansprüchen 1 und 2, enthaltend Verbindungen der allgemeinen Formel I, worin
R¹ für Methyl,
R² für R³,
R³ für 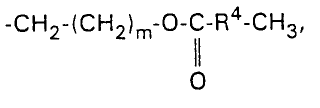 m für die Zahl 1,
R⁴ für einen Alkyl- oder Alkenylrest mit 16 C-Atomen steht.

4. Mischung nach den Ansprüchen 1 bis 3, enthaltend Verbindungen der allgemeinen Formel I, worin
R¹ für -CH₂-CH₂-OH,
R² für R³,
R³ für 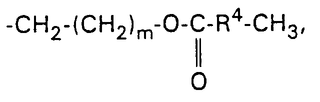 m für die Zahl 1,
R⁴ für einen Alkyl- oder Alkenylrest mit 16 C-Atomen steht.

5. Mischung nach den Ansprüchen 1 bis 4, dadurch gekennzeichnet, daß sie bis zu 50 Gew.-%, vorzugsweise zwischen 10 und 40 Gew.-% Verbindungen der allgemeinen Formel I, und bis zu 90 Gew.-%, vorzugsweise zwischen 55 und 85 Gew.-% Lösungsmittel auf Basis natürlicher oder synthetischer Öle und gegebenenfalls zusätzlich oder anstelle der Lösungsmittel langkettige Fettsäuren der Formel II oder deren Alkylester sowie gegebenenfalls weitere Hilfsmittel bis zu 30 Gew.-% enthalten.

6. Verwendung der Mischung nach den Ansprüchen 1 bis 6 zur Herstellung von nichtstaubenden und nichtzusammenbackenden Düngemitteln.

7. Düngemittel, dadurch gekennzeichnet, daß sie mit der Mischung nach den Ansprüchen 1 bis 6 behandelt worden sind.
